Europäisches Patentamt

European Patent Office

Office européen des brevets

(19)

(11) Veröffentlichungsnummer : **0 119 386**
**B1**

(12) **EUROPÄISCHE PATENTSCHRIFT**

(45) Veröffentlichungstag der Patentschrift :
30.10.85

(51) Int. Cl.⁴ : **C 07 C149/243**

(21) Anmeldenummer : **84100180.3**

(22) Anmeldetag : **10.01.84**

(54) **Verfahren zur Gewinnung von D,L-Homocystin.**

(30) Priorität : **18.03.83 DE 3309762**

(43) Veröffentlichungstag der Anmeldung :
**26.09.84 Patentblatt 84/39**

(45) Bekanntmachung des Hinweises auf die Patenterteilung : **30.10.85 Patentblatt 85/44**

(84) Benannte Vertragsstaaten :
**AT BE CH FR GB IT LI LU NL SE**

(56) Entgegenhaltungen :
US-A- 2 406 362
CHEMICAL ABSTRACTS, Band 70, Nr. 19, 12. Mai 1969, Seite 13, Nr. 84330p, Columbus, Ohio, USA; D. CAVALLINI et al.: "Copper-catalyzed oxidation of cysteine to cystine"

(73) Patentinhaber : **Degussa Aktiengesellschaft**
**Weissfrauenstrasse 9**
**D-6000 Frankfurt am Main 1 (DE)**

(72) Erfinder : **Karrenbauer, Michael, Dr. Dipl.-Chem.**
**Lindenstrasse 10**
**D-6458 Rodenbach (DE)**
Erfinder : **Kleemann, Axel, Dr. Dipl.-Chem.**
**Greifenhagenstrasse 25**
**D-6450 Hanau 9 (DE)**
Erfinder : **Lüssling, Theodor, Dr. Dipl.-Chem.**
**Zum Purren 5**
**D-7750 Konstanz Litzelstetten (DE)**
Erfinder : **Schäfer, Fritz, Dr. Dipl.-Chem.**
**Uhlandstrasse 25**
**D-7750 Konstanz (DE)**

Jouve, 18, rue St-Denis, 75001 Paris, France

## Beschreibung

Gegenstand der Erfindung ist ein Verfahren zur Gewinnung von D, L-Homocystin durch Oxidation des Dinatriumsalzes von D, L-Homocystein.

D, L-Homocystin ist als Zusatz bei der Herstellung von Heimtiernahrung von Interesse.

Es ist zwar bekannt, Mercaptane zu den entsprechenden Disulfiden zu oxidieren. Im Falle des D, L-Homocysteins werden jedoch nur bei Einhaltung ganz bestimmter Bedingungen hohe Ausbeuten an dem gewünschten D, L-Homocystin erhalten.

Das erfindungsgemäße Verfahren ist dadurch gekennzeichnet, daß man in eine wäßrige Lösung des Dinatriumsalzes von D, L-Homocystein mit einer Konzentration zwischen 0,8 und 1,2 Mol/l und einem Anfangs-pH zwischen 7,0 und 8,0 in Gegenwart von katalytischen Mengen an $Fe^{3+}$- oder $Cu^{2+}$-Ionen solange molekularen Sauerstoff einleitet, bis kein Ansteigen des pH mehr festzustellen ist, und anschließend unter Rühren den pH auf etwa 5,3 einstellt.

Unter diesen Reaktionsbedingungen wird überraschenderweise das gewünschte D, L-Homocystin in Ausbeuten von über 80 % erhalten. Setzt man dagegen die wäßrige Lösung des Dinatriumsalzes von D, L-Homocystein mit einer Konzentration von weniger als 0,8 Mol/l oder mehr als 1,2 Mol/l oder mit einem Anfangs-pH von weniger als 7,0 oder mehr als 8,0 ein, werden deutlich geringere Ausbeuten erhalten.

Die als Ausgangsmaterial dienenden wäßrigen Lösungen des Dinatriumsalzes von D, L-Homocystein können in bekannter Weise durch Entmethylierung von D, L-Methionin mittels Natrium in flüssigem Ammoniak, anschließendes Abdampfen des Ammoniaks und Aufnehmen des verbleibenden Rückstandes in einer geeigneten. Menge Wasser hergestellt werden.

Die Einstellung des Anfangs-pH auf einen Wert zwischen 7,0 und 8,0 erfolgt zweckmäßigerweise mit einer wäßrigen Mineralsäure, vorzugsweise Salzsäure.

Die Oxidation erfolgt in Gegenwart von katalytischen Mengen, beispielsweise 50 bis 500 mg pro Mol eingesetzten Dinatriumsalzes von D, L-Homocystein, an $Fe^{3+}$- oder $Cu^{2+}$-Ionen.

Das Ende der Oxidationsreaktion ist daran zu erkennen, daß bei weiterem Einleiten von molekularem Sauerstoff keine Erhöhung des pH mehr eintritt. Dies ist im allgemeinen nach etwa 3 bis 4 Stunden der Fall.

Nach beendeter Oxidation wird unter Rühren der pH des Reaktionsgemisches, zweckmäßigerweise mit einer wässrigen Mineralsäure, vorzugsweise Salzsäure, auf etwa 5,3 eingestellt. Es ist vorteilhaft, wenn die Einstellung des pH bei erhöhter Temperatur, beispielsweise 50 °C, vorgenommen wird und anschließend das Reaktionsgemisch unter Rühren langsam, beispielsweise im Verlauf einer Stunde, auf Raumtemperatur abgekühlt wird. In einer besonders vorteilhaften kristallinen Form fällt das D, L-Homocystin dann an, wenn die Rührgeschwindigkeit während der Ausfällung so bemessen wird, daß das D, L-Homocystin gerade noch in Suspension gehalten wird. Es kann dann besonders leicht durch Filtration oder Zentrifugation abgetrennt werden.

Die Erfindung wird durch die nachfolgenden Beispiele und Vergleichsversuche näher erläutert. Prozentangaben bedeuten, wenn nicht anders angegeben, Gewichtsprozente.

Beispiel 1

13,95 g durch Entmethylierung von D, L-Methionin hergestelltes Dinatriumsalz von D, L-Homocystein werden in Wasser gelöst und mit Salzsäure versetzt, so daß eine 0,8-molare Lösung mit einem pH von 7,0 entsteht. Diese Lösung wird dann mit 77 mg Eisen- (III)- Sulfat versetzt. Anschließend werden innerhalb von 3 Stunden bei 25 °C 99,6 $dm^3$ Sauerstoff eingeleitet (der große Sauerstoffüberschuß erklärt sich durch die nicht berücksichtigten erheblichen Durchschlagsverluste). Die Reaktionsmischung wird dann auf 50 °C erwärmt und unter Rühren mit 10 %iger Salzsäure auf einen pH von 5,3 eingestellt. Unter Rühren wird innerhalb einer Stunde auf Raumtemperatur abgekühlt. Das ausgefallene D, L-Homocystin wird abgesaugt, mit 100 ml Wasser von 80 °C nachgewaschen und im Vakuumtrockenschrank bis zur Gewichtskonstanz getrocknet. Die Ausbeute an D, L-Homocystin beträgt 9,2 g, entsprechend 88 % der Theorie.

Beispiel 2

Das Beispiel 1 wird wiederholt mit dem Unterschied, daß das Dinatriumsalz von D, L-Homocystein mit soviel Wasser und Salzsäure versetzt wird, daß eine 0,8-molare Lösung mit einem pH von 8,0 entsteht. Die Ausbeute an D, L-Homocystin beträgt 9,6 g, entsprechend 92 % der Theorie.

Vergleichsversuch 1

Das Beispiel 1 wird wiederholt mit dem Unterschied, daß das Dinatriumsalz von D, L-Homocystin mit soviel Wasser und Salzsäure versetzt wird, daß eine 0,8-molare Lösung mit einem pH von 9,0 entsteht. Die Ausbeute an D, L-Homocystin beträgt 3,2 g, entsprechend 30,6 % der Theorie.

Beispiel 3

20,85 g durch Entmethylierung von D, L-Methionin hergestelltes Dinatriumsalz von D, L-Homocystein werden in Wasser gelöst und mit Salzsäure versetzt, so daß eine 1,2-molare Lösung mit einem pH von 8,0 entsteht. Anschließend wird analog Beispiel 1 Sauerstoff eingeleitet und das Reaktionsgemisch aufgearbeitet. Die Ausbeute an D, L-Homocystin beträgt 14,83 g, entsprechend 95 % der Theorie.

Vergleichsversuch 2

Das Beispiel 3 wird wiederholt mit dem Unterschied, daß das Dinatriumsalz des D, L-Homocysteins mit soviel Wasser und Salzsäure versetzt wird, daß eine 1,4-molare Lösung mit einem pH von 8,0 entsteht. Die Ausbeute an D, L-Homocystin beträgt 7,49 g, entsprechend 48 % der Theorie.

Vergleichsversuch 3

Das Beispiel 3 wird wiederholt mit dem Unterschied, daß das Dinatriumsalz des D, L-Homocysteins mit soviel Wasser und Salzsäure versetzt wird, daß eine 0,4-molare Lösung mit einem pH von 8,0 entsteht. Die Ausbeute an D, L-Homocystin beträgt 8,96 g, entsprechend 57,4 % der Theorie.

**Patentanspruch**

Verfahren zur Gewinnung von D, L-Homocystin durch Oxidation des Dinatriumsalzes von D, L-Homocystein, dadurch gekennzeichnet, daß man in eine wäßrige Lösung des Dinatriumsalzes von D,˙ L-Homocystein mit einer Konzentration zwischen 0,8 und 1,2 Mol/l und einem Anfangs-pH zwischen 7,0 und 8,0 in Gegenwart von katalytischen Mengen an $Fe^{3+}$- oder $Cu^{2+}$-Ionen solange molekularen Sauerstoff einleitet, bis kein Ansteigen des pH mehr festzustellen ist, und anschließend unter Rühren den pH auf etwa 5,3 einstellt.

**Claim**

A process for recovering D, L-homocystine by oxidizing the disodium salt of D, L-homocysteine, characterised in that molecular oxygen is introduced into an aqueous solution of the disodium salt of D, L-homocysteine with a concentration from 0.8 to 1.2 mol/l and a starting pH from 7.0 to 8.0 in the presence of catalytic quantities of $Fe^{3+}$- or $Cu^{2+}$-ions until no further increase in the pH can be determined, and the pH is subsequently adjusted with stirring to about 5.3.

**Revendication**

Procédé pour l'obtention de D, L-Homocystine par oxydation du sel disodique de la D, L-Homocystéine, caractérisé en ce que l'on introduit, dans une solution aqueuse de sel disodique de D, L-Homocystéine dont la concentration se situe entre 0,8 et 1,2 mol/l, et le pH initial est situé entre 7 et 8, en présence de quantités catalytiques d'ions $Fe^3$ ou $Cu^2$, de l'oxygène moléculaire, jusqu'à ce qu'on ne puisse plus observer d'élévation du pH, et ensuite, ajuste le pH à environ 5,3 tout en agitant.